# EUROPÄISCHE PATENTANMELDUNG

(11) **EP 3 000 499 A1**
(43) Veröffentlichungstag der Anmeldung: **30.03.2016**
(21) Anmeldenummer: 15185884.2
(22) Anmeldetag: 18.09.2015
(51) Int. Cl.: A61M 11/00, A61M 16/16, F24F 6/12, B05B 17/00, B60H 3/00, B60H 1/00, A61M 15/00, B60H 3/02, B64D 11/06

(54) **VORRICHTUNG ZUR BEFEUCHTUNG DER ATEMLUFT EINES BENUTZERS UND DESSEN VERWENDUNG**

(30) Priorität: 26.09.2014 DE 102014114009
(71) Anmelder: Kern, Stefan, 63820 Elsenfeld (DE)
(72) Erfinder: Kern, Stefan, 63820 Elsenfeld (DE)
(74) Vertreter: Pöhner, Wilfried Anton

(57) **Zusammenfassung**

Die Erfindung betrifft eine Vorrichtung (100) zur Befeuchtung der Atemluft eines Benutzers umfassend eine Verneblereinheit (101) mit einer Einfüllöffnung (102), einem Reservoir für ein Fluid (103), einem Deckel (104), einer Austrittsöffnung (105), einem piezoelektrischen Ultraschallschwinger und einer Membran (106) und einer elektronischen Treibstufe (110) mit einem Spannungswandler und/oder einer Endstufe, mit einem Ein-/Ausschalter (111) und mit einem Stromkabel (120). Des Weiteren betrifft die Erfindung die Verwendung dieser Vorrichtung in einem Fahrzeug.

## Beschreibung

Die Erfindung betrifft eine Vorrichtung zur Befeuchtung der Atemluft eines Benutzers umfassend eine Verneblereinheit mit einer Einfüllöffnung, einem Reservoir für ein Fluid, einem Deckel, einer Austrittsöffnung, einem piezoelektrischen Ultraschallschwinger und einer Membran und einer elektronischen Treibstufe mit einem Spannungswandler und/oder einer Endstufe, mit einem Ein-/Ausschalter und mit einem Stromkabel. Des Weiteren betrifft die Erfindung die Verwendung dieser Vorrichtung in einem Fahrzeug.

Der Mensch hält sich mindestens zwei Drittel seiner Lebenszeit in geschlossenen Räumen auf. Allerdings wird der Luftqualität in Räumen immer noch viel zu wenig Beachtung geschenkt, obwohl diese einen entscheidenden Einfluss auf dessen Gesundheit, sowie zur Steigerung des körperlichen und geistigen Leistungsvermögens hat. Sie trägt zudem zu einem angenehmen Raumklima bei.

Ein für den Menschen angenehmes Raumklima hat eine Temperatur von ca. 22°C und eine relative Luftfeuchtigkeit zwischen 40 - 60 %. Dieser Wert wird jedoch vor allem in geschlossenen, klimatisierten, zu stark belüfteten oder gut beheizten Räumen oft unterschritten, was zur Folge hat, dass die Schleimhäute der Atemwege austrocknen, ihre Immunabwehr sinkt und sich ihre Fähigkeit zum Stoffaustausch reduziert. Die Schleimhäute sind auf ihre hohe Feuchtigkeit zur Erhaltung ihrer Funktionen angewiesen und verfügen jedoch nur über einen geringen Verdunstungsschutz. Bei zu niedriger relativer Luftfeuchtigkeit wird die Schleimhaut, beispielsweise die Nasen-, Mund- und Rachenschleimhaut, anfällig für Hautreizungen bzw. - rötungen oder gar Hautentzündungen, so dass u.a. das Risiko für Erkältungserkrankungen und das Auftreten von Nasenbluten steigt. Dies ist insbesondere im Winter und in vielen klimatisierten Bereichen der Fall, beispielsweise in Fahrzeugen und/oder im Flugzeug.

Um das Risiko des Austrocknens der Schleimhaut zu reduzieren, ist es ratsam, dem überall dort, wo diese Gefahr aufgrund zu niedriger relativer Luftfeuchtigkeit besteht, effektiv entgegenzuwirken, beispielsweise durch die Verwendung eines Raumluftbefeuchters. Derartige Systeme verdampfen oder vernebeln Wasser, um auf diese Weise die Feuchtigkeit in der Luft in einem geschlossenen Raum zu erhöhen. Allerdings eignen sich derartige Raumluftbefeuchter nicht für alle Räume. So wäre beispielsweise in Flugzeugen eine erhebliche Menge an Wasser nötig, um die relative Luftfeuchtigkeit im Flugzeuginnenraum nennenswert über eine zentrale Versorgung zu erhöhen. Aus Gewichtsgründen, zur Vermeidung von Kondensation und Korrosion an den Kabinenwänden und den daraus resultierenden elektrischen Fehlfunktionen, sowie aus hygienischen Gründen ist es allerdings ausgeschlossen, dass eine derartige zentrale Versorgung stattfindet. In den Zuführkanälen können sich Totvolumina bilden, in denen sich Bakterien, Viren, Schimmelpilze o.ä. ansiedeln können. Ähnliche Probleme können auch in Fahrzeugen auftreten.

Um diese Probleme zu vermeiden, empfiehlt es sich, beispielsweise lediglich die Luftqualität in einem lokal beschränkten Bereich durch Erhöhung der Feuchtigkeit der Luft zu verbessern, beispielsweise indem eine Vorrichtung in unmittelbarer Nähe vor einem Benutzer, wie einem Passagier, Begleitpersonal, Fahrer und/oder Beifahrer, angebracht wird. Auf diese Weise verbessert sich die Luftqualität lediglich lokal für den Benutzer, ohne dass die relative Luftfeuchtigkeit des gesamten geschlossenen Raumes durch die lokal eingebrachte Feuchtigkeit signifikant ansteigt.

Die bisher verfügbaren Systeme zur Lösung des oben beschriebenen Problems sind meist sehr groß und schwer, sowie aufgrund ihrer Vielzahl vorhandener Anschlüsse oftmals unhandlich und sperrig. Kleinere Vorrichtungen weisen hingegen oft den Nachteil auf, dass deren Laufzeit speziell bei Batterie/Akku-betriebenen Vorrichtungen stark eingeschränkt ist.

Eine kleine Vorrichtung zum Befeuchten der Atemluft eines Benutzers ist beispielsweise in der Offenlegungsschrift WO 2013/164253 A1 beschrieben. Diese betrifft einen Zerstäuber, der mittels einer universellen Datenleitung mit Energie versorgt und über einen angeschlossenen Computer mittels einer speziellen Software individuell gesteuert wird. Die Datenleitung ist bevorzugt über einen USB-Anschluss an den Computer angeschlossen. Mittels dieses Zerstäubers ist es daher möglich, die Luftqualität des Benutzers in einem lokal beschränkten Bereich zu verbessern, indem dieser permanent und/oder über einen kurzen Zeitraum aktiviert wird. Allerdings ist für dessen Funktionsweise ein Computer mit der entsprechenden darauf installierten Software nötig, so dass sich dieser Zerstäuber für einen mobilen Einsatz im Flugzeug und/oder Fahrzeug nicht unbedingt eignet.

Für den mobilen Einsatz ist es notwendig, dass die Vorrichtung handlich, klein und ohne störende Anschlüsse versehen ist, so dass sich der Benutzer möglichst frei bewegen kann. In der Offenlegungsschrift DE 10 2010 009 666 A1 ist beispielsweise eine derartige mobile Vorrichtung zum Befeuchten der Atemluft eines Benutzers beschrieben. Diese ist wie ein Headset ausgestaltet und erlaubt die Modifizierung der Luftqualität mittels einer Austrittsöffnung unmittelbar vor dessen Atemöffnungen. Aus dieser Austrittsöffnung tritt permanent befeuchtete Atemluft aus, welche zusätzlich mit Duftstoffen angereichert sein kann. Das Headset weist zudem zusätzliche Funktionen auf; so kann beispielsweise mittels dieses Headsets Musik gehört, telefoniert und auch diktiert werden.

Das beschriebene Headset hat allerdings den Nachteil, dass es vom Benutzer immer getragen werden muss und dass die Versorgung mit befeuchteter Atemluft permanent erfolgt. Es ist daher verständlich, dass ein Headset bei langer Tragedauer, beispielsweise auf einer langen Reise im Reisebus, im Fernverkehrs- oder Nahverkehrszug, im Flugzeug oder auf einer Fähre, vom Benutzer als störend und unkomfortabel empfunden werden kann.

Es besteht daher ein großer Bedarf an einer Vorrichtung, welche möglichst klein, leicht und komfortabel in der Handhabung ist, damit ein Benutzer insbesondere auf längeren Reisen in einem lokal beschränkten Bereich mit befeuchteter Atemluft versorgt werden kann, ohne die Feuchtigkeit der Luft im gesamten Raum zu erhöhen. Um dies zu realisieren, ist es vorteilhaft, dass der Benutzer bei Bedarf und/oder persönlichem Bedürfnis individuell mit befeuchteter Atemluft versorgt wird. Eine solche intermittierende Befeuchtung der Atemluft hat einerseits den Vorteil, dass die Handlichkeit und die Handhabung der Vorrichtung erhöht wird, da diese nicht permanent getragen bzw. gehalten werden muss. Es konnte zudem gezeigt werden, dass die respiratorische Dehydrierung eines Benutzers in geschlossenen Räumen bereits dadurch verhindert wird, dass dieser lediglich intermittierend mit befeuchteter Atemluft versorgt wird.

Aufgabe der Erfindung ist es also, eine leichte und handliche Vorrichtung zur Verhinderung der respiratorischen Dehydrierung eines Benutzers in geschlossenen Räumen bereitzustellen, ohne die relative Luftfeuchtigkeit des Raumes zu erhöhen, um so die oben genannten Schwierigkeiten zu überwinden.

Diese Aufgabe wird durch die Vorrichtung mit den Merkmalen des unabhängigen Anspruchs insbesondere dadurch gelöst, dass die Verbindung der elektronischen Treibstufe und der Verneblereinheit lösbar ist. Vorteilhafte Weiterbildungen der Erfindung, welche einzeln oder in Kombination realisierbar sind, sind in den Unteransprüchen dargestellt.

Der Begriff "Befeuchtung der Atemluft" betrifft die Verbesserung der Atemluftqualität eines Benutzers, indem bevorzugt die Feuchtigkeit der Atemluft erhöht wird. Ein Fachmann kennt diverse Möglichkeiten, dies zu realisieren. Beispielsweise wird durch das Aufstellen von Zimmerpflanzen, eines handelsüblichen Verneblers, Verdunsters, Zerstäubers und/oder Sprudlers die relative Luftfeuchtigkeit im gesamten Raum erhöht. Dies ist für den angegeben Einsatzbereich der erfindungsgemäßen Vorrichtung allerdings mit erheblichen Nachteilen verbunden. Besonders bevorzugt erfolgt diese Erhöhung daher in einem lokal beschränkten Bereich vor den Atemöffnungen des Benutzers, d.h. vor dessen Nase und/oder Mund. Am meisten bevorzugt positioniert der Benutzer die Vorrichtung unmittelbar, d.h. in geringem Abstand, vor seinen Atemöffnungen. Die Befeuchtung der Atemluft kann auch als Inhalation erfolgen. Zudem kann ein Mundstück auf die Austrittsöffnung der erfindungsgemäßen Vorrichtung aufgesetzt werden, um eine intensivere Befeuchtung zu erreichen. Da lediglich in einem kleinen Bereich vor den Atemöffnungen des Benutzers die Atemluft befeuchtet wird, kommt es somit zu keiner signifikanten Erhöhung der relativen Luftfeuchtigkeit im gesamten Raum.

Bevorzugt erfolgt die Befeuchtung der Atemluft benutzerindividuell, indem der Benutzer die Austrittsöffnung oder das Mundstück der Verneblereinheit vor wenigstens einer Atemöffnung individuell positioniert. Für die wirksame Befeuchtung der Atemluft ist es ja lediglich ausschlaggebend, dass der Benutzer diese vor seine Atemöffnungen hält und dass die Austrittsöffnung in Richtung der Atemöffnung ausgerichtet ist. Folglich ist jede mögliche Position der Vorrichtung relativ zu den Atemöffnungen umfasst, wie über, unter, links, rechts und/oder davor.

Die Befeuchtung der Atemluft erfolgt über einen beliebigen Zeitraum, d. h. sie kann permanent oder auch über einen kürzeren Zeitraum stattfinden. Vorteilhafterweise wurde herausgefunden, dass bereits eine Befeuchtung über einen kürzeren Zeitraum mit positiven gesundheitlichen Effekten für den Benutzer verbunden ist. Um derartige positive gesundheitliche Effekte zu erzielen ist es daher ausreichend, wenn die Befeuchtung der Atemluft lediglich intermittierend erfolgt, indem der Benutzer bei Bedarf, wie bei medizinischer Notwendigkeit, und/oder persönlichem Bedürfnis den Ein-/Ausschalter der elektronischen Treibstufe betätigt. Daher ist es nicht nötig, dass die erfindungsgemäße Vorrichtung dauerhaft von dem Benutzer gehalten und/oder permanent körperfest angebracht ist. Vorteilhafterweise kann die Vorrichtung bei Nicht-Benutzung einfach zur Seite gelegt und/oder in einer Tasche verstaut werden.

Der Begriff "Verneblereinheit" bezeichnet eine Einheit zur Herstellung und zur Abgabe eines Aerosols und/oder eines feinen Nebels. Bevorzugt wird mittels eines piezoelektrischen Ultraschallschwingers und einer Membran ein feiner Nebel hergestellt. Eine derartige Einheit kann aus jedem Material bestehen und ist so ausgebildet, dass ein Hohlraum entsteht, in welchen ein Fluid eingefüllt bzw. eingegeben wird. Die Verneblereinheit umfasst im allgemeinsten Zustand eine Einfüllöffnung, ein Reservoir für ein Fluid, einen Deckel, eine Austrittsöffnung, einen piezoelektrischen Ultraschallschwinger und eine Membran. Zusätzlich kann die Verneblereinheit eine eingebaute Kontrollleuchte, insbesondere eine Betriebszustandskontrollleuchte, zur optischen Erfassung des Betriebszustandes der Vorrichtung umfassen. Eine derartige Kontrollleuchte ist bevorzugt eine farbige LED.

Die Verneblereinheit kann auch mehr als nur eine Einfüllöffnung und eine entsprechende Anzahl an Reservoiren für die Fluide umfassen, in welche dieselben und/oder verschiedene Fluide eingegeben werden können. Im Falle mehrerer Einfüllöffnungen kann lediglich ein Deckel zur Abdeckung aller Einfüllöffnungen oder entsprechend mehrere Deckel umfasst sein.

Bevorzugt ist die Verneblereinheit derart ausgebildet, dass der umfasste piezoelektrische Ultraschallschwinger und/oder die Membran an einer Grenz- und/oder Oberfläche des eingefüllten bzw. eingegebenen Fluides angrenzt. Der Begriff "Membran" ist bekannt und betrifft ein durchlässiges Material, welches bevorzugt synthetischer oder biologischer Herkunft ist. Bevorzugt ist das Gewebe ein wasserdurchlässiges Textil-Netzgewebe, noch mehr bevorzugt ein Mesh-Gewebe bzw. eine Mesh-Membran. Ein derartiges Gewebe umfasst Poren, welche sich bevorzugt nach außen hin verjüngen.

Mittels eines piezoelektrischen Ultraschallschwingers und einer Membran wird das Fluid in der Verneblereinheit vernebelt. Bevorzugt erfolgt die Verneblung mittels Umwandlung der in der Verneblereinheit angelegten elektrischen Spannung in eine mechanische Schwingung im Ultraschallbereich, wobei aus dem Fluid, dessen Grenz- und/oder Oberfläche mit dem piezoelektrischen Ultraschallschwinger und/oder der Membran in Kontakt tritt, feine Tröpfchen gebildet und abgegeben werden. Diese Tröpfchen sind unterschiedlich groß. Bevorzugt sind diese jedoch so fein, dass sie längere Zeit in der Luft schweben und in ihrer Mehrheit einen Nebel erzeugen können. Durch den Nebel, welcher dem Benutzer über eine Austrittsöffnung zugeleitet wird, wird die Atemluft befeuchtet. Es versteht sich, dass für die Funktion der Verneblereinheit Wechselspannung benötigt wird.

Der Begriff "Einfüllöffnung" bezeichnet eine Öffnung in der Verneblereinheit, durch welche ein Fluid in einen Hohlraum eingefüllt bzw. eingegeben wird. Bevorzugt wird ein Fluid über die Einfüllöffnung in das Reservoir der Verneblereinheit eingefüllt bzw. eingegeben.

Bevorzugt umfasst die Verneblereinheit in Höhe der Einfüllöffnung einen Anschluss, mit welchem wenigstens ein zusätzliches Element, insbesondere ein Deckel und/oder eine Kapsel bzw. Patrone, mit der Verneblereinheit verbunden werden kann. Beispielsweise ist dieser Anschluss eine Steck-, Schraub-, Bajonett- oder Schnappverbindung. Bevorzugt ist dieser eine Steckverbindung, so dass wenigstens ein zusätzliches Element mit der Verneblereinheit verbunden werden kann. Besonders bevorzugt ist für das Lösen dieses Anschlusses kein Werkzeug notwendig.

Dieser Anschluss kann derart ausgestaltet sein, dass die Verbindung wieder lösbar ist. Alternativ wird mit diesem Anschluss eine unlösbare Verbindung erreicht. Weiterhin bevorzugt schließt dieser Anschluss das Reservoir dicht ab. Ein Fachmann weiß dies zu realisieren. Verständlicherweise können die einzelnen Ausgestaltungen beliebig miteinander kombiniert werden, so kann beispielsweise mittels des Anschlusses ein Deckel und/oder eine Kapsel bzw. Patrone lösbar und dicht mit der Einfüllöffnung verbunden werden.

Die Einfüllöffnung ist mit einem Deckel, welcher aus jedem Material sein kann, verschlossen. Der Deckel ist bevorzugt derart ausgebildet, dass dieser die Einfüllöffnung und/oder das Reservoir der Verneblereinheit dicht abschließt. Alternativ schließt der Deckel die Einfüllöffnung mit der mit ihr verbundenen Kapsel bzw. Patrone dicht ab. Daher dient der verschlossene Deckel zudem als Auslaufstopp, um zu verhindern, dass das einfüllte bzw. eingegebene Fluid aus dem Reservoir der Verneblereinheit ausläuft.

Der Deckel umfasst keine Öffnungen. Alternativ kann der Deckel allerdings auch wenigstens eine Öffnung umfassen, um das Einfüllen bzw. Eingeben des Fluides über den Deckel zu ermöglichen.

Der Begriff "Austrittsöffnung" bezeichnet eine Öffnung in der Verneblereinheit, durch welche ein vernebeltes Fluid austreten und so einem Benutzer zugeleitet werden kann. Bevorzugt umfasst die Verneblereinheit wenigstens eine Austrittsöffnung, über welche das vernebelte Fluid aus der Verneblereinheit austritt. Noch mehr bevorzugt umfasst die Austrittsöffnung ein Verbindungssystem, mit welchem wenigstens ein zusätzliches Element mit der Austrittsöffnung verbunden werden kann. Beispielsweise ist dieses Verbindungssystem eine Steck-, Schraub-, Bajonett- oder Schnappverbindung. Bevorzugt ist es eine Steckverbindung, so dass wenigstens ein zusätzliches Element mit der Austrittsöffnung verbunden werden kann. Besonders bevorzugt ist für das Lösen dieses Verbindungssystems kein Werkzeug notwendig.

Dieses Verbindungssystem kann derart ausgestaltet sein, dass die Verbindung wieder lösbar ist. Alternativ wird mit diesem Verbindungssystem eine unlösbare Verbindung erreicht. So kann beispielsweise mittels des Verbindungssystems ein austauschbares Mundstück mit der Austrittsöffnung verbunden werden. Das Mundstück ist bevorzugt derart ausgestaltet, dass es mit der Austrittsöffnung gas- und wasserdicht verbunden ist. Bevorzugt ist das Mundstück lösbar mit der Austrittsöffnung verbunden. Alternativ ist das Mundstück unlösbar mit der Austrittsöffnung verbunden.

Das Mundstück bzw. die Verneblereinheit mit dem unlösbar verbundenen Mundstück ist aus hygienischen Gründen austauschbar. Das Mundstück ist des Weiteren bevorzugt derart ausgestaltet, dass es sich für die Atmung mit dem Mund und/oder der Nase eignet. Dann atmet der Benutzer die befeuchtete Atemluft über den Mund und/oder die Nase ein, um so tiefere Atemwege zu versorgen. Verständlicherweise können die einzelnen Ausgestaltungen beliebig miteinander kombiniert werden.

Der Begriff "Fluid" bezeichnet jedes Gas und jede Flüssigkeit, welche sich zum Vernebeln eignet. Das Fluid umfasst wenigstens einen Inhaltsstoff, bevorzugt gasförmiges Wasser, flüssiges Wasser, Sauerstoff, Aerosol, einen Duftstoff und/oder eine pharmazeutisch wirksame Substanz. Das Fluid kann auch eine Mischung von wenigstens zwei der genannten Inhaltsstoffe umfassen. Zudem ist bevorzugt, dass wenigstens zwei verschiedene Fluide verwendet werden, welche dieselben und/oder verschiedene Inhaltsstoffe umfassen. Bevorzugt ist das Fluid Wasser, besonders bevorzugt sehr weiches Wasser, um Mineralablagerungen in und an der Vorrichtung zu vermeiden. Noch mehr bevorzugt ist das Fluid Wasser mit wenigstens einem zusätzlichen Inhaltsstoff.

Alternativ ist das Fluid in einer Kapsel bzw. Patrone eingebracht, welche mittels eines Anschlusses mit der Einfüllöffnung verbunden ist. Bevorzugt sind wenigstens zwei verschiedene Fluide umfasst, welche dieselben und/oder verschiedene Inhaltsstoffe haben und in der Kapsel bzw. Patrone voneinander separiert sind. Zur Erleichterung der Verbindung umfasst die Kapsel bzw. Patrone eine Perforation als Sollbruchstelle. Alternativ umfasst die Einfüllöffnung, der Deckel und/oder das Reservoir eine Einrichtung zur erleichterten Öffnung der Kapsel bzw. Patrone, so dass diese beim Einsetzen perforiert. Der Deckel kann dann derart ausgestaltet sein, dass dieser die Einfüllöffnung mit der verbundenen Kapsel bzw. Patrone dicht abschließt. Die beschriebenen Ausgestaltungen können zudem beliebig miteinander kombiniert werden.

Der Begriff "elektronische Treibstufe" bezeichnet eine Baugruppe, welche die zugeführte elektrische Energie in für die Verneblereinheit benötigte Strom- bzw. Spannungswerte umwandelt. Der Aufbau dieser Baugruppe ist von der angelegten Stromversorgung abhängig. So kann Gleichstrom durch einen Spannungswandler, insbesondere einem Gleichrichter, aus Wechselstrom gebildet werden. Mittels eines Gleichspannungswandlers kann die eine am Eingang zugeführte Gleichspannung in eine Gleichspannung mit höherem, niedrigerem oder invertiertem Spannungsniveau umgewandelt werden. Im umgekehrten Fall kann aus Gleichstrom mit Hilfe eines Spannungswandlers, insbesondere ein Wechselrichter, Wechselstrom erzeugt werden. Da die Verneblereinheit für ihre Funktionsweise Wechselspannung benötigt, wandelt die elektronische Treibstufe die zugeführte elektrische Energie bevorzugt in Wechselspannung um.

Die elektrische Treibstufe umfasst somit Komponenten, welche für die Bedienung der Verneblereinheit notwendig sind und welche notwendig sind, um die über das Stromkabel zugeführte elektrische Energie in Wechselspannung umzuwandeln. Der Fachmann weiß, wie dies realisiert wird.

Im allgemeinsten Fall umfasst die elektronische Treibstufe daher ein Bedienelement, eine Baugruppe zur Wandlung und/oder Erhöhung bzw. Verringerung der Spannung, eine Anschlussmöglichkeit für ein Stromkabel und/oder eine Stromversorgung und eine Einrichtung zur Zuleitung der elektrischen Energie zum piezoelektrischen Ultraschallschwinger. Bei angelegtem Gleichstrom ist diese Baugruppe zur Wandlung der Spannung ein Spannungswandler, insbesondere ein Wechselrichter. Bei angelegtem Wechselstrom ist kein Gleichrichter notwendig, da der piezoelektrische Ultraschallschwinger Wechselstrom benötigt. In diesem Fall ist die Baugruppe zur Erhöhung bzw. Verringerung der Spannung ein Trafo, ein Spannungsverstärker und/oder eine Endstufe, insbesondere eine HF-Endstufe. Natürlich müssen ein Spannungsverstärker und/oder eine Endstufe, insbesondere eine HF-Endstufe, auch in einer elektronischen Treibstufe mit angelegter Gleichspannung vorhanden sein. Mittels der oben genannten Komponenten wird es ermöglicht, dass die Verneblereinheit über die elektronische Treibstufe und ein daran angeschlossenes Stromkabel bzw. eine Stromversorgung angetrieben wird.

Bevorzugt erhält die elektronische Treibstufe Energie über einen Universal Serial Bus (USB)-Anschluss, so dass diese neben der angeschlossenen Stromversorgung über USB ein Bedienelement, einen Spannungswandler, insbesondere einen Wechselrichter, und eine Einrichtung zur Zuleitung der elektrischen Energie zum piezoelektrischen Ultraschallschwinger umfasst. Zusätzlich kann die elektronische Treibstufe eine Kontrollleuchte und/oder einen Timer aufweisen.

Des Weiteren bevorzugt ist die elektronische Treibstufe mit einer lösbaren Verbindung mit der Verneblereinheit verbunden. Beispielsweise ist diese Verbindung eine Steck-, Schraub-, Bajonett- oder Schnappverbindung. Bevorzugt ist die Verbindung eine Steckverbindung, so dass die elektronische Treibstufe einfach auf die Verneblereinheit auf- bzw. eingesteckt wird. Besonders bevorzugt ist für das Lösen dieser Verbindung kein Werkzeug notwendig. Am meisten bevorzugt ist die lösbare Verbindung eine Steckverbindung mit Universal Serial Bus (USB).

Eine derartige lösbare Verbindung ist wichtig, um die kontaminationsanfällige Verneblereinheit aus hygienischen Gründen in regelmäßigen Abständen schnell und einfach austauschen zu können. Die elektronische Treibstufe bedarf aus hygienischen Gründen keines Austausches.

Der Begriff "Stromkabel" bezeichnet ein Kabel, welches elektrische Energie in ausreichender Leistung übertragen kann und die Vorrichtung mit der Stromversorgung verbindet. Die Stromversorgung befindet sich in Reichweite des Benutzers, insbesondere in der Nähe seines Sitzes. Bevorzugt umfasst das Kabel wenigstens zwei Leitungen. In dem Fall, dass die von der Energieversorgung bereitgestellte Spannung nicht zu der von der elektronischen Treibstufe benötigten Spannung passt, umfasst das Stromkabel zusätzlich eine Umspannung, wie einen Wechselrichter und/oder einen Trafo.

Das Stromkabel ist derart ausgestaltet, dass es eine Geräteseite und eine Versorgungsseite aufweist. Bevorzugt weist das Stromkabel geräte- und versorgungsseitig einen Schutz vor dem Ab- und/oder Umknicken auf, wie beispielsweise die Gehäuseeinführung.

Bevorzugt ist die Geräteseite des Stromkabels unlösbar mit der elektronischen Treibstufe verbunden und umfasst eine Gehäuseeinführung zur Zugentlastung und/oder zum Schutz des Kabels gegen Ab- und/oder Umknicken.

Alternativ ist die Geräteseite des Stromkabels mittels einer lösbaren Verbindung mit der elektronischen Treibstufe verbunden. Bevorzugt ist diese Verbindung eine Steck-, Schraub-, Bajonett- oder Schnappverbindung. Noch mehr bevorzugt ist die lösbare Verbindung eine Steckverbindung mit einem standardisierten Anschluss, so dass das Stromkabel geräteseitig einfach mit der elektronischen Treibstufe verbunden werden kann. Besonders bevorzugt ist für das Lösen dieser Verbindung kein Werkzeug notwendig.

Die Geräteseite des Stromkabels weist einen Gerätestecker auf. Die Steckerstandards unterscheiden sich hauptsächlich nach dem Spannungsniveau oder der Spannungsart und der erwarteten zu übertragenden Stromstärke. Bevorzugt ist der Steckerstandard der Norm IEC 60320. Des Weiteren bevorzugt ist der Steckerstandard ein Universal Serial Bus (USB). Zudem bevorzugt ist der Steckerstandard eine Bordspannungs- oder Autosteckdose nach DIN ISO 4165 und/oder ein Zigarettenanzünder nach der Norm SAE J563.

Vergleichbares gilt für die Versorgungsseite des Stromkabels. Bei der Auswahl des Standards der Steckverbindung bedarf es zusätzlich der Berücksichtigung regionaler Verbreitungen der entsprechenden Standards am beabsichtigten Einsatzort. Beispielsweise kann das Stromkabel zur Energieversorgung mit einem Computer, Akkupack, einer Batterie oder einem elektrischen Versorgungsnetz verbunden werden.

Bevorzugt entspricht das Stromkabel dem Universal Serial Bus (USB)-Standard, da dieser international sehr verbreitet ist.

Vorteilhafterweise kann die Vorrichtung mittels der beschriebenen lösbaren Verbindungen, welche sich alle lösen lassen, zerlegt werden. Bevorzugt ist die Vorrichtung mittels der lösbaren Verbindung in die aus hygienischen Gründen auszutauschende Verneblereinheit und die elektronische Treibstufe zerlegbar. Noch mehr bevorzugt ist die Vorrichtung mittels der beschriebenen lösbaren Verbindungen in die Verneblereinheit, die elektronische Treibstufe und das Stromkabel mit einem standardisierten Anschluss zerlegbar, indem die Verbindung der Verneblereinheit und der elektronischen Treibstufe und die Verbindung der elektronischen Treibstufe und des Stromkabels lösbar ist. Auf diese Weise wird erreicht, dass die erfindungsgemäße Vorrichtung klein, leicht und handlich ist.

Der Begriff "Ein-/Ausschalter" betrifft ein Bedienelement, mit welchem ein Benutzer einen Eingriff auf einen Energie- und/oder Informationsfluss erlangen kann. Bevorzugt ist der Ein-/Ausschalter ein Taster oder Schalter. Der Ein-/Ausschalter kann auch als getrennte Schalter ausgeführt sein.

Falls der Ein-/Ausschalter ein Schalter ist, beginnt die Befeuchtung der Atemluft bei Betätigung des Ein-/Ausschalters und endet bei erneuter Betätigung des Ein-/Ausschalters. Alternativ ist der Ein-/Ausschalter ein Taster, d.h. die Atemluft wird solange befeuchtet, wie der Ein-/Ausschalter betätigt wird. Bei Beendigung der Betätigung des Ein-/Ausschalters endet auch die Befeuchtung.

Das bedeutet, dass die Verneblung des Fluides mittels des Ein-/Ausschalters gestartet und/oder beendet wird. Alternativ wird die Verneblung automatisch beendet, beispielsweise indem die elektronische Treibstufe zusätzlich einen Timer umfasst. Mittels des Timer wird es ermöglicht, dass die Befeuchtung der Atemluft automatisch, d.h. nach einem geräteseitig eingestellten Zeitintervall, beendet wird, um eine lediglich intermittierende Befeuchtung der Atemluft sicherzustellen, ohne, dass der Benutzer den Ein-/Ausschalter erneut betätigen bzw. loslassen muss. Auf diese Weise wird sichergestellt, dass die Befeuchtung der Atemluft nicht zu lange andauert.

Der Begriff "Atemluft" bezeichnet jedes Gasgemisch, das für die Atmung verwendet werden kann, wie beispielsweise Umgebungsluft.

Der Begriff "Benutzer" bezeichnet eine Person, die ein Hilfsmittel zur Erzielung eines Vorteils oder eines Nutzens verwendet.

Der Begriff "Fahrzeug" bezeichnet ein mobiles Verkehrsmittel für den Transport von Gütern, Werkzeugen und/oder Personen. Bevorzugt ist dieses ein Verkehrsmittel für Personen, mit welchem diese zu Lande, zu Wasser und/oder in der Luft fortbewegt werden können. Bevorzugt ist das Fahrzeug ein Landfahrzeug, ein Wasserfahrzeug oder ein Luftfahrzeug. Besonders bevorzugt ist das Fahrzeug ein Massentransportmittel für Fernverkehrsstrecken, wie beispielsweise ein Reisebus, ein Fernverkehrs- oder Nahverkehrszug, ein Flugzeug oder eine Fähre.

Es wird davon ausgegangen, dass die Definitionen und Ausführungen der oben genannten Begriffe für alle in dieser Beschreibung im Folgenden beschriebenen Aspekte gelten, sofern nichts anders angegeben ist.

Die Erfindung betrifft des Weiteren die Verwendung einer Vorrichtung zum Befeuchten der Atemluft eines Benutzers in einem Fahrzeug. Vorteilhafte Weiterbildungen der Erfindung, welche einzeln oder in Kombination realisierbar sind, sind in den Unteransprüchen dargestellt.

Die Verwendung der Vorrichtung zur Befeuchtung der Atemluft eines Benutzers erfolgt derart, dass ein Stromkabel der Vorrichtung mit einer Stromversorgung verbunden und diese durch den Benutzer aufgenommen, insbesondere in die Hand genommen, wird. Bevorzugt wird das Stromkabel in Reichweite des Benutzers mit einer Stromversorgung, insbesondere einer in der Nähe des Sitzes verfügbaren Stromversorgung, verbunden, so dass dieser im Sitzen und ohne den Sitz verlassen zu müssen, die Vorrichtung bedienen kann. Die Vorrichtung wird in geringem Abstand vor einer Atemöffnung des Benutzers, insbesondere dem Mund und/oder der Nase, positioniert, wobei es wichtig ist, dass die Austrittsöffnung in Richtung der Atemöffnung ausgerichtet ist.

Im Normalzustand ist die Vorrichtung ausgeschaltet. Mit Betätigen eines Ein-/Ausschalters wird die Vorrichtung eingeschaltet und die Verneblung wird gestartet, wobei die Verneblung eines Fluides in der Verneblereinheit mittels eines piezoelektrischen Ultraschallschwingers und einer Membran erfolgt. Das Fluid kann bereits in der Verneblereinheit eingefüllt bzw. eingegeben sein. Nach erneutem Betätigen des Ein-/Ausschalters wird die Verneblung beendet und die Vorrichtung wird ausgeschaltet. Der Ein-/Ausschalter kann dabei als Schalter oder Taster ausgestaltet sein. Alternativ endet die Verneblung automatisch, d.h. nach einem geräteseitig eingestellten Zeitintervall und/oder mittels eines Timer. In diesem Fall schaltet sich die Vorrichtung nach dem Beenden der Verneblung automatisch ab.

Nach dem Ausschalten der Vorrichtung wird diese durch den Benutzer abgelegt.

Der Betriebszustand der Vorrichtung ist mittels der eingebauten Kontrollleuchte, insbesondere einer Betriebszustandskontrollleuchte, leicht kontrollierbar.

Über die Austrittsöffnung tritt das vernebelte Fluides aus der Verneblereinheit aus, welches vom Benutzer eingeatmet wird. Bevorzugt erfolgt die Einatmung des Benutzers synchron zum Austritt des vernebelten Fluids.

Die Verwendung der Vorrichtung erfolgt in einem Fahrzeug, noch mehr in einem Massentransportmittel für Fernverkehrsstrecken. Am meisten bevorzugt erfolgt die Verwendung jedoch in einem Flugzeug.

Weitere Einzelheiten, Merkmale und Vorteile der Erfindung ergeben sich aus der nachfolgenden Beschreibung des bevorzugten Ausführungsbeispiels in Verbindung mit den Unteransprüchen. Hierbei können die jeweiligen Merkmale für sich alleine oder zu mehreren in Kombination miteinander verwirklicht sein. Die Erfindung ist nicht auf das Ausführungsbeispiel beschränkt. Das Ausführungsbeispiel ist in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:
- Figur 1A - 1D: die erfindungsgemäße Vorrichtung in verschiedenen Ansichten bzw. Schnitten;
- Figur 2A - 2B: die erfindungsgemäße Vorrichtung in verschiedenen Ansichten mit einem Stromkabel mit USB-Anschluss.

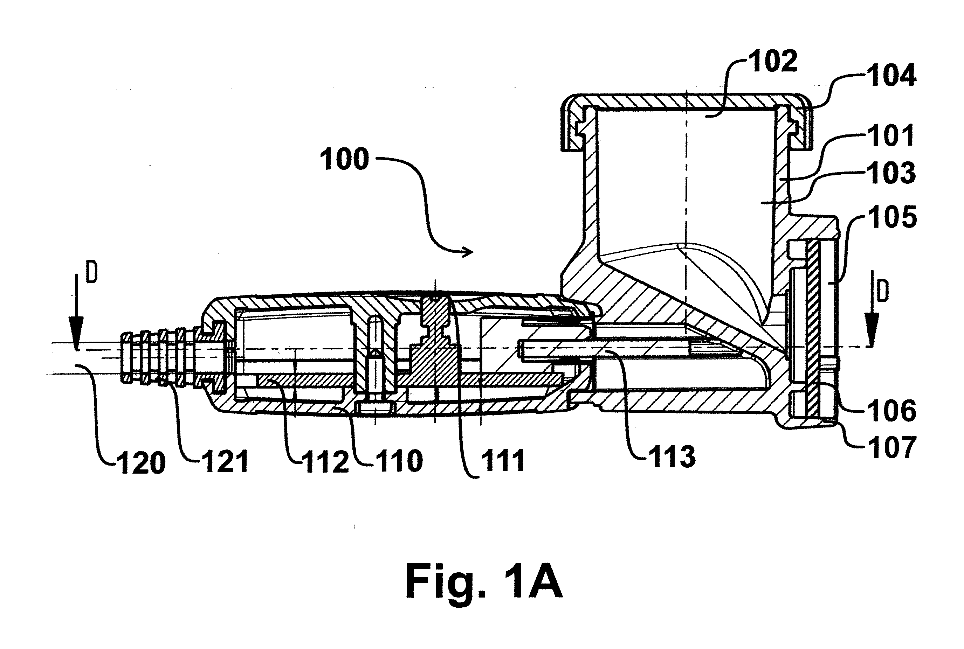

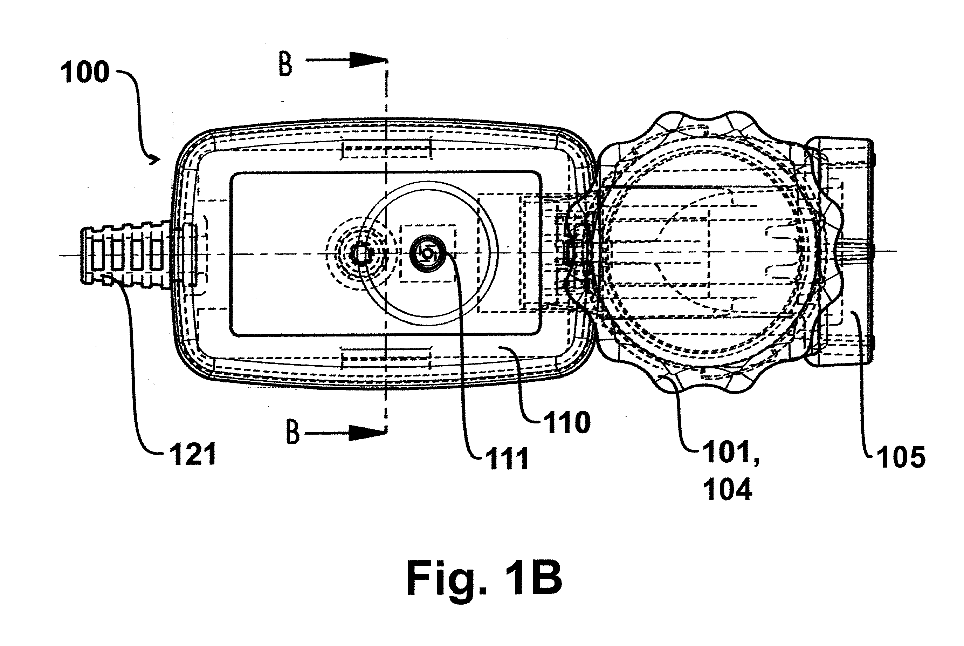

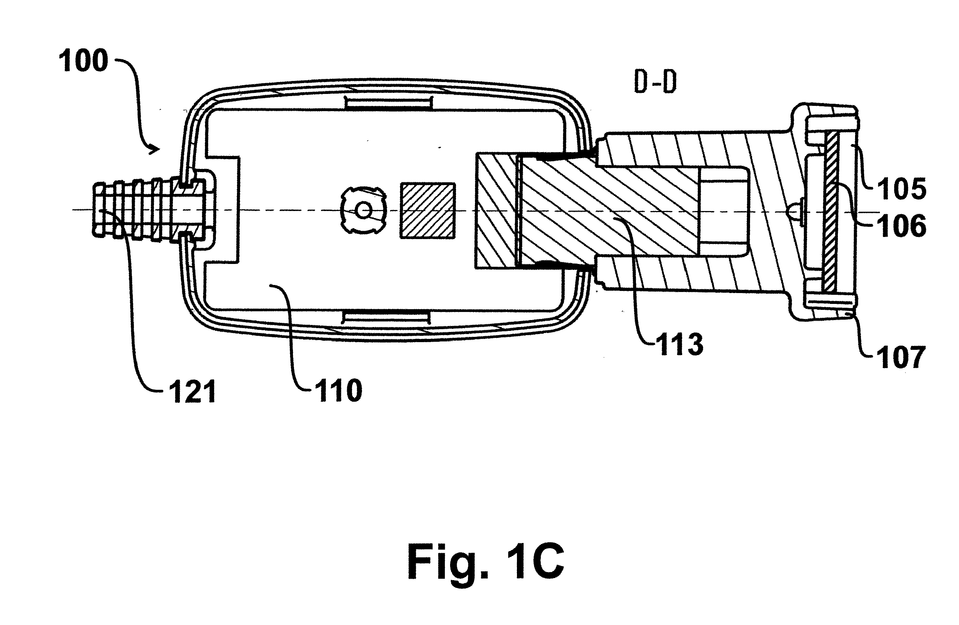

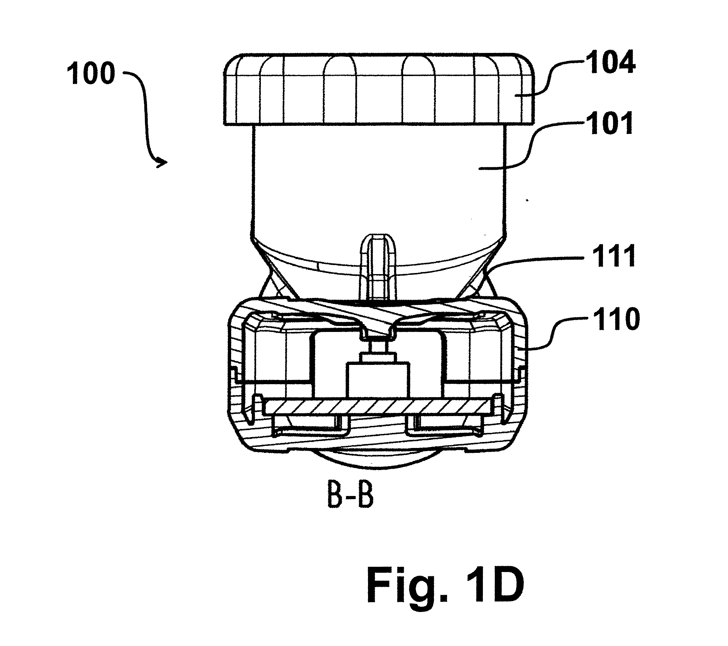

In den Figuren 1A bis 1D ist eine erfindungsgemäße Vorrichtung (100) in schematischer Darstellung in der Vorderansicht (Fig. 1A) und in der Draufsicht (Fig. 1 B), sowie in der Horizontalschnittebene D - D (Fig. 1C) und in der Längsschnittebene B - B (Fig. 1D) gezeigt. Gleichzeitig dienen die Figuren 1A bis 1D zur Erläuterung eines möglichen Ausführungsbeispiels der Vorrichtung zum Befeuchten der Atemluft eines Benutzers.

Die Vorrichtung (100) umfasst die Verneblereinheit (101) mit der Einfüllöffnung (102), dem Reservoir für ein Fluid (103), dem Deckel (104) zum gas- und/oder wasserdichten Verschließen der Einfüllöffnung (102) und der Austrittsöffnung (105). Nach Entfernen des Deckels (104) kann das Fluid über die Einfüllöffnung (102) in das Reservoir (103) eingefüllt bzw. eingegeben werden. Alternativ kann die Einfüllöffnung (102) auch derart ausgestaltet sein, dass eine Kapsel bzw. Patrone mit dieser verbunden wird, welche ein zu verdampfendes Fluid enthält. Der verschlossene Deckel (104) dient zudem als Auslaufstopp, um zu verhindern, dass das eingefüllte bzw. eingegebene Fluid aus der Verneblereinheit (101) ausläuft. Der mittels dem piezoelektrischen Ultraschallschwinger und der Membran (106) hergestellte feine Nebel wird dem Benutzer über die Austrittsöffnung (105) zugeführt. Die Membran (106) ist im Wesentlichen senkrecht zur Austrittsöffnung (105) ausgerichtet. Mit der Austrittsöffnung (105) kann alternativ ein austauschbares Mundstück verbunden werden.

Die Vorrichtung (100) umfasst des Weiteren die elektronische Treibstufe (110) mit dem Ein-/Ausschalter (111), wobei die elektronische Treibstufe (110) auf die Verneblereinheit (101) mittels der lösbaren Verbindung (113) ein- bzw. aufgesteckt ist. Der von der elektronischen Treibstufe (110) umfasste Ein-/Ausschalter (111) ist als Schalter oder Taster ausgestaltet und beginnt bzw. beendet die Befeuchtung der Atemluft. Die elektronische Treibstufe (110) ist unlösbar mit dem Stromkabel (120) verbunden und weist die Gehäuseeinführung (121) als Ab- und/oder Umknickschutz des Stromkabels auf.

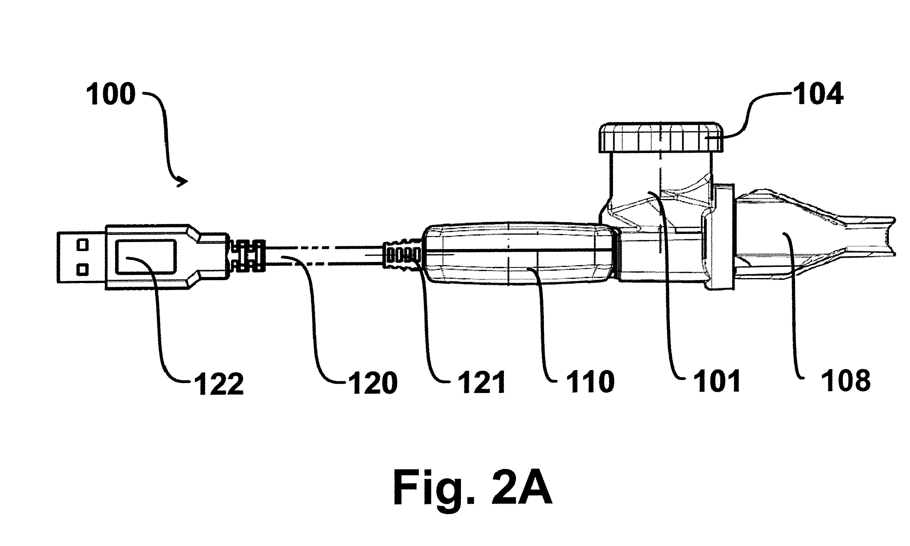

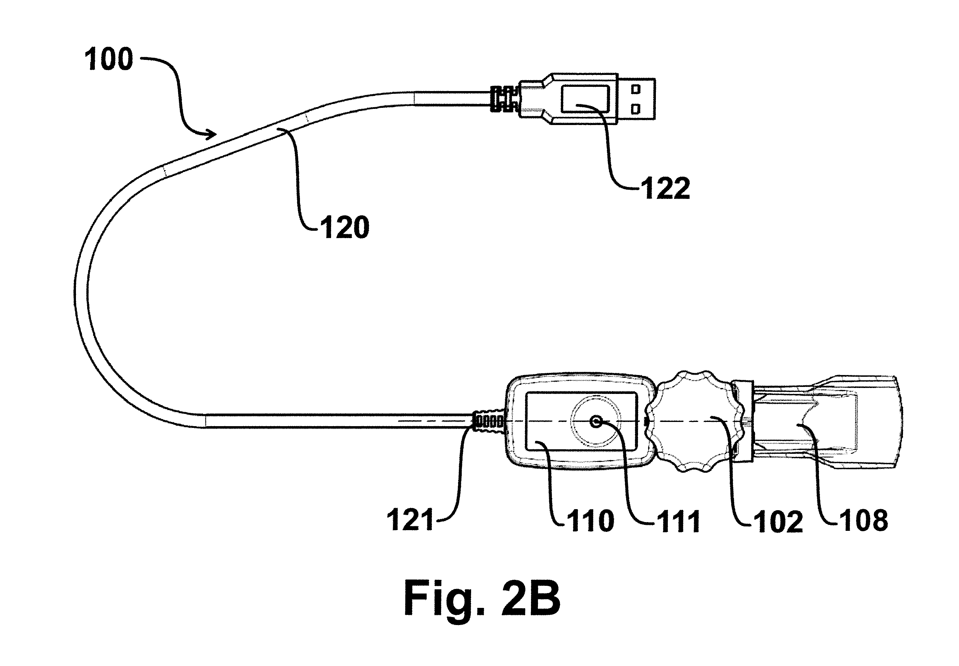

In den Figuren 2A bis 2B ist eine erfindungsgemäße Vorrichtung (100) mit dem Stromkabel (120) mit USB-Anschluss (122) in schematischer Darstellung in der Vorderansicht (Fig. 2A) und in der Draufsicht (Fig. 2B) gezeigt. Die Figuren 2A und 2B dienen zur Erläuterung eines möglichen Ausführungsbeispiels der erfindungsgemäßen Vorrichtung.

Die Vorrichtung (100) umfasst die Verneblereinheit (101) mit der Einfüllöffnung (102), dem Deckel (104) und dem mit einer Austrittsöffnung verbundenen Mundstück (108). Die Verneblereinheit (101) ist mit der elektronischen Treibstufe (110) verbunden, an welche sich ein Stromkabel (120) mit der Gehäuseeinführung (121) und dem USB-Stecker (122) anschließt. Die elektronische Treibstufe (110) umfasst den Ein-/Ausschalter (111) für den Beginn bzw. die Beendigung der Befeuchtung der Atemluft.

### Bezugszeichenliste

- 100: erfindungsgemäße Vorrichtung
- 101: Verneblereinheit
- 102: Einfüllöffnung
- 103: Reservoir für das Fluid
- 104: Deckel
- 105: Austrittsöffnung
- 106: Membran
- 107: Stutzen für das Mundstück
- 108: Mundstück

- 110: elektronische Treibstufe
- 111: Ein-/Ausschalter
- 112: Treiberelektronik mit Spannungswandler und HF-Endstufe
- 113: USB-Verbindung

- 120: Stromkabel
- 121: Gehäuseeinführung
- 122: USB-Stecker des Stromkabels

## Patentansprüche

1. Vorrichtung zur Befeuchtung der Atemluft eines Benutzers umfassend
- eine Verneblereinheit (101) mit einer Einfüllöffnung (102), einem Reservoir für ein Fluid (103), einem Deckel (104), einer Austrittsöffnung (105), einem piezoelektrischen Ultraschallschwinger und einer Membran (106); und
- einer elektronischen Treibstufe (110) mit einem Spannungswandler und/oder einer Endstufe, mit einem Ein-/Ausschalter (111) und mit einem Stromkabel (120)
**gekennzeichnet durch** eine lösbare Verbindung (113) der elektronischen Treibstufe (110) mit der Verneblereinheit (101).

2. Vorrichtung nach dem vorhergehenden Anspruch, **dadurch gekennzeichnet, dass** das Stromkabel (120) dem Universal Serial Bus-Standard entspricht.

3. Vorrichtung nach dem vorhergehenden Anspruch, **dadurch gekennzeichnet, dass** der Ein-/Ausschalter (111) ein Schalter oder Taster ist.

4. Vorrichtung nach dem vorhergehenden Anspruch, **dadurch gekennzeichnet, dass** die elektrische Treibstufe (110) zusätzlich einen Timer zur automatischen Beendung der Verneblung umfasst.

5. Vorrichtung nach einem der vorhergehenden Ansprüche,
**dadurch gekennzeichnet, dass** das Fluid Wasser umfasst.

6. Vorrichtung nach einem der vorhergehenden Ansprüche,
**dadurch gekennzeichnet, dass** das Fluid in einer Kapsel bzw. Patrone eingebracht ist, welche mittels eines Verbindungssystems mit der Einfüllöffnung (102) verbunden ist.

7. Vorrichtung nach einem der vorhergehenden Ansprüche,
**dadurch gekennzeichnet, dass** der Deckel (104) die Einfüllöffnung (102) dicht abschließt.

8. Vorrichtung nach einem der vorhergehenden Ansprüche,
**dadurch gekennzeichnet, dass** mit der Austrittsöffnung (105) mittels eines Verbindungssystems (107) ein Mundstück (108) verbunden ist.

9. Vorrichtung nach einem der vorhergehenden Ansprüche,
**dadurch gekennzeichnet, dass** die Membran (106) eine Mesh-Membran ist.

10. Verwendung einer Vorrichtung nach einem der vorhergehenden Ansprüche zur Befeuchtung der Atemluft eines Benutzers in einem Fahrzeug umfassend die Schritte:
- Verbinden eines Stromkabels (120) mit einer Stromversorgung;
- Aufnehmen der Vorrichtung durch den Benutzer;
- Positionieren der Vorrichtung in geringem Abstand vor einer Atemöffnung des Benutzers, wobei die Austrittsöffnung (105) in Richtung der Atemöffnung ausgerichtet ist;
- Betätigen eines Ein-/Ausschalters (111), wobei die Vorrichtung eingeschaltet und die Verneblung eines Fluides in der Verneblereinheit (101) mittels eines piezoelektrischen Ultraschallschwingers und einer Membran (106) gestartet wird;
- Austreten des vernebelten Fluides aus der Verneblereinheit (101) über eine Austrittsöffnung (105);
- Einatmen des vernebelten Fluides;
- Betätigen des Ein-/Ausschalters (111), wobei die Verneblung des Fluides beendet und die Vorrichtung ausgeschaltet wird; und
- Ablegen der Vorrichtung durch den Benutzer.

11. Verwendung nach dem vorhergehenden Anspruch, **dadurch gekennzeichnet, dass** das Fahrzeug ein Massentransportmittel für Fernverkehrsstrecken ist.

12. Verwendung nach dem vorhergehenden Anspruch, **dadurch gekennzeichnet, dass** das Fahrzeug ein Flugzeug ist.

13. Verwendung nach dem vorhergehenden Anspruch, **dadurch gekennzeichnet, dass** der Benutzer synchron zum Austritt des vernebelten Fluids einatmet.

14. Verwendung nach dem vorhergehenden Anspruch, **dadurch gekennzeichnet, dass** das Stromkabel (120) in Reichweite des Benutzers mit einer Stromversorgung verbunden wird.
